(19) Europäisches Patentamt
European Patent Office
Office européen des brevets

(11) Veröffentlichungsnummer : **0 036 554**
**B1**

(12) # EUROPÄISCHE PATENTSCHRIFT

(45) Veröffentlichungstag der Patentschrift : **05.06.85**

(51) Int. Cl.⁴ : **G 01 N 33/64**

(21) Anmeldenummer : **81101744.1**

(22) Anmeldetag : **10.03.81**

(54) **Diagnostisches Mittel zum Nachweis von Keton-Körpern.**

(30) Priorität : **22.03.80 DE 3011168**

(43) Veröffentlichungstag der Anmeldung : **30.09.81 Patentblatt 81/39**

(45) Bekanntmachung des Hinweises auf die Patenterteilung : **05.06.85 Patentblatt 85/23**

(84) Benannte Vertragsstaaten : **AT BE CH DE FR GB IT LI LU NL SE**

(56) Entgegenhaltungen :
**DE-A- 2 733 426**
**DE-B- 1 256 920**
**DE-B- 2 158 125**
**Chemical Abstracts Band 92, Nr. 9 3. März 1980 Columbus, Ohio, USA F. WADA et al. "New applications of crown ethers. I. Primary alkylamine-water-crown ether system as a hydroxide ion source in the reduction with carbonylhydridoferrate anion" Seite 596, Spalte 1, Abstract Nr. 75523f**

(73) Patentinhaber : **BEHRINGWERKE AKTIENGESELLSCHAFT**
**Postfach 1140**
**D-3550 Marburg/Lahn (DE)**

(72) Erfinder : **Kohl, Helmut, Dr.**
**Goethestrasse 32**
**D-3552 Wetter (DE)**

(74) Vertreter : **Meyer-Dulheuer, Karl-Hermann, Dr. et al**
**HOECHST Aktiengesellschaft Zentrale Patentabteilung Postfach 80 03 20**
**D-6230 Frankfurt/Main 80 (DE)**

**EP 0 036 554 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die Erfindung betrifft ein diagnostisches Mittel zum Nachweis von Keton-Körpern in Flüssigkeiten.

Der Nachweis von Keton-Körpern im Urin ist für die Erkennung und Überwachung von Diabetikern von großer Bedeutung. Die empfindliche und schnelle Kontrolle wird heute mit Schnelldiagnostika, beispielsweise Urin-Teststreifen, durchgeführt. Das Prinzip des Keton-Nachweises ist jeweils dar der Legal'schen Probe. Die Konzentration des durch die Umsetzung eines Keton-Körpers mit Natriumnitroprussid in alkalischem Milieu entstehenden tiefvioletten Farbstoffes dient als Maß für die Menge des vorhandenen Keton-Körpers.

Zur Herstellung von Keton-Teststreifen werden entsprechend DE-PS 12 56 920 Papiere mit einem wässrigen Puffer und einer Aminosäure getränkt. In einem zweiten Imprägniergang muß dann der Indikator, das Natriumnitroprussid, in einem organischen Lösungsmittel auf das vorimprägnierte Papier appliziert werden. Dieses Verfahren ist notwendig, da der Indikator im Bereich über pH 7 labil ist und sich zu braunen Folgeprodukten zersetzt, wenn er mit Spuren von Wasser, beispielsweise Luftfeuchtigkeit, in Berührung kommt. Diese braune Grundfarbe macht einen empfindlichen Nachweis unmöglich. Die Zersetzung kann aber auch so weit gehen, daß auf dem Testpapier kein Indikator mehr vorhanden ist.

Zur Stabilisierung werden nach der DE-PS 12 56 920 der basische Puffer und das Natriumnitroprussid durch eine Schutzschicht aus einer folienbildenden Chemikalie getrennt. Gemäß dieser Lehre werden zwar relativ stabile Papiere erhalten, ihre Reaktionsgeschwindigkeit und Empfindlichkeit entsprechen aber nicht mehr den Anforderungen. Entsprechend den Patentschriften DE-PS 21 58 125 und DE-PS 26 05 221 werden die mit dem basischen Puffer vorimprägnierten Papiere mit einer Lösung von Natrtiumnitroprussid in Methanol und einem mit diesem mischbaren organischen Lösungsmittel getränkt. Die so hergestellten Papiere sind spröde und neigen bei der Konfektionierung zum Brechen, so daß teilweise aufwendige Reaktionsträger verwendet werden müssen (DE-PS 21 58 124). Die Stabilität dieser Papiere ist Schwankungen unterworfen.

Auch bei diesen Papieren wird der stabilisierende Effekt auf ein Trennmittel zurückgeführt. Dieses Trennmittel ist das jeweilige mit Methanol mischbare Lösungsmittel (DE-PS 26 05 221). Entsprechend der DE-OS 27 33 426 wird dagegen der basische Puffer zunächst mit einer organischen Säure, beispielsweise Ölsäure, abgedeckt.

In einem dritten Imprägnierschritt wird dann der Indikator aufgetragen. Auch diese Papiere sind nur bedingt lagerstabil und müssen beispielsweise bei einer relativen Luftfeuchtigkeit von unter 40 % verarbeitet werden, da sie sich sonst sofort braun verfärben. Bedingt durch die dritte Imprägnierung sind die Papiere spröde und brechen leicht; aufgrund der großen Salzmengen auf den Papieren lassen sie sich auch schlecht mittels der üblichen Klebetechniken auf einer Trägerfolie fixieren.

Der vorliegenden Erfindung lag infolgedessen die Aufgabe zugrunde, einen Stabilisator für ein diagnostisches Mittel zum Nachweis von Keton-Körpern in Flüssigkeiten zu finden, mit dessen Hilfe ohne besondere Vorsichtsmaßnahmen Keton-Testpapiere herstellbar sind, die leicht zu verarbeiten und lagerstabil sind und beim Gebrauch die notwendige Empfindlichkeit besitzen.

Die Aufgabe wird erfindungsgemäß dadurch gelöst, daß das diagnostische Mittel zum Nachweis von Keton-Körpern in Flüssigkeiten aus einer Trägermatrix besteht, die imprägniert wurde mit einem Puffer, einer Aminosäure und Natriumnitroprussid in einem organischen Lösemittel, und das gekennzeichnet ist durch einen Gehalt an einer Verbindung der Formel I

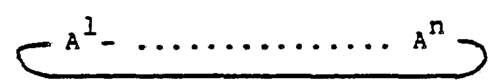 (I)

in welcher n eine Zahl von 2 bis 10 ist, und worin ein A-Glied

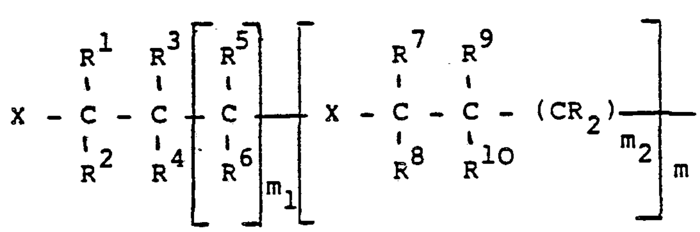

bedeutet, worin

X ein Sauerstoffatom, ein Schwefelatom oder NH,

R bis $R^{10}$ je ein Wasserstoffatom,

$m_1$ und $m_2$ eine Zahl von 0 bis 4 und

m die Zahl 0 oder 1, wobei jedoch mindestens vier X-Reste in der Verbindung vorhanden sein müssen, ist und außerdem

$R^3$, $R^4$, $R^9$ und $R^{10}$ $C_{1-3}$-Alkyl

$R^1$ und $R^3$ oder $R^7$ und $R^9$ zusammen $CH_2CH_2CH_2CH_2$ oder CH=CH—CH=CH und —$R^2$ und —$R^4$ oder

—$R^8$ und —$R^{10}$ zusammen eine Bindung und $R^5$, $R^6$, $R^7$ und $R^8$ zusammen CH—CH, wobei $m_1$ = 1 ist, (sowie $R^1$ und $R^2$ sowie $R_2$ mit den entsprechenden R-Resten der nicht gezeichneten Nachbarglieder A, wobei $m_2$ = 1 ist, zusammen CH—CH) sein können oder eine Verbindung der Formel II ist

$$N \begin{array}{l} \diagup (CH_2)_n - X - (CH_2)_{n} - Y - (CH_2)_m \diagdown \\ - (CH_2)_{n_1} - X_1 - (CH_2)_{n_1} - Y_1 - (CH_2)_{m_1} - \\ \diagdown (CH_2)_{n_2} - X_2 - (CH_2)_{n_2} - Y_2 - (CH_2)_{m_2} \diagup \end{array} N \qquad (II)$$

in welcher

n, $n_1$ und $n_2$ die Zahlen 2 oder 3,

m, $m_1$ und $m_2$ eine Zahl von 0 bis 3,

X, $X_1$, $X_2$, Y, $Y_1$ und $Y_2$ ein Sauerstoff- oder Schwefelatom oder

—Y—, —$Y_1$— und —$Y_2$— eine Bindung, in welchem Fall m, $m_1$ beziehungsweise $m_2$ = 0 ist,

bedeutet.

Die stabilisierende Wirkung dieser Verbindungen war überraschend. Erstaunlich ist auch, daß die Stabilisierung auch mit solchen Verbindungen der Formel I oder II eintritt, die als hygroskopisch angesehen werden. Ihre positive Wirkung ist dann am größten, wenn sie zusammen mit dem Natriumnitroprussid aufgetragen werden ; sie tritt aber auch ein, wenn wasserlösliche Verbindungen der Formel I oder II mit dem basischen Puffer auf die Trägermatrix aufgetragen werden. Die in den fertigen Papieren vorhandene Menge an Stabilisator sollte der jeweiligen Menge an Indikator angepaßt werden, obwohl die absolute Menge nicht kritisch ist. So kann das molare Verhältnis von Stabilisator zu Indikator zwischen 0,1 und 15 Mol auf 1 Mol Indikator liegen. Prinzipiell ist die Menge des Stabilisators nur durch dessen Löslichkeit im jeweils verwendeten Lösemittel limitiert.

Vorteilhaft sind Verbindungen der Formel I, worin X ein Sauerstoffatom oder NH, $m_1$ und $m_2$ die Zahl 0 oder 1, $R^5$ und $R^6$, falls $m_1$ gleich 1 ist, je ein Wasserstoffatom bedeuten und die übrigen Symbole die oben angegebene Bedeutung haben.

Bevorzugt sind Verbindungen der Formel I und den Bedeutungen der Symbole

n : die Ziffer 2 oder 3

X : ein Sauerstoffatom

$m_1$, $m_2$ : Null

$R^1$-$R^4$ : ein Wasserstoffatom

$R^7$-$R^{10}$ : ein Wasserstoffatom

$R^1$ und $R^3$ : zusammen CH=CH—CH=CH oder $CH_2$—$CH_2$—$CH_2$—$CH_2$

$R^2$ und $R^4$ : zusammen eine Bindung

m : 0 oder 1.

Besonders bevorzugt sind die folgenden Verbindungen, wobei für einige die Bedeutungen der Symbole angegeben sind, durch deren Einsetzen in die jeweilige allgemeine Formel diese Verbindungen erhalten werden :

12-crown-4

15-crown-5 (Formel I : n = 3 ; in allen Gliedern ist X Sauerstoff, $m_1$ = $m_2$ = Null oder $R^1$-$R^4$ und $R^7$-$R^{10}$ Wasserstoff ; in 2 Gliedern ist m = 1 und in einem m = 0)

18-crown-6 (Formel I : n = 3 ; in allen A-Gliedern ist X ein Sauerstoff, $m_1$ = $m_2$ = Null, m = 1, $R^1$-$R^4$ und $R^7$-$R^{10}$ sind Wasserstoffatome)

Dibenzo-18-crown-6 (Formel I : n = 3 ; in allen A-Gliedern ist X ein Sauerstoffatom, $m_1$ = $m_2$ = 0, m = 1 ; in $A^1$ ist $R^1$ und $R^3$ zusammen CH=CH—CH=CH und —$R^2$ und —$R^4$ zusammen eine Bindung und $R^7$-$R^{10}$ sind Wasserstoffatome ; in $A^2$ sind $R^1$-$R^4$ Wasserstoffatome, $R^7$ und $R^9$ zusammen CH=CH—CH=CH und —$R^8$ und —$R^{10}$ zusammen eine Bindung ; in $A^3$ sind $R^1$-$R^4$ und $R^7$-$R^{10}$ Wasserstoffatome)

Dicyclohexano-18-crown-6

1,4,7,10,13,16-Hexaaza-cyclooctadecan-trisulfat

Verbindungen der Kryptofix(R)

Reihe von Merck, wie zum Beispiel Kryptofix(R) 222, 211 und 221 (Verbindungen der Formel II, worin n, $n_1$, $n_2$, m, $m_1$ und $m_2$ die Zahl 2 und X, $X_1$, $X_2$, Y, $Y_1$ und $Y_2$ ein Sauerstoffatom bzw. n, $n_1$, $n_2$ und $m_2$ die Zahl 2, m und $m_1$ Null und X, $X_1$, $X_2$ und $Y_2$ ein Sauerstoffatom und —Y— und —$Y_1$— eine Bindung beziehungsweise n, $n_1$, $n_2$, $m_1$ und $m_2$ die Zahl 2, m Null und X, $X_1$, $X_2$, $Y_1$ und $Y_2$ ein Sauerstoffatom und —Y— eine Bindung bedeuten)

Besonders geeignet sind stabile Verbindungen wie Dibenzo-18-crown-6 oder 18-crown-6.

Bevorzugt wird ein Molverhältnis von 0,5 Mol bis 1,5 Mol Stabilisator zu 1 Mol Indikator, wenn der Stabilisator in einem organischen Lösemittel aufgetragen wird. Bei der Applikation gemeinsam mit dem Puffer sollte dagen die molekulare Menge des Stabilisators zwischen 7 Mol und 12 Mol liegen.

Es hat sich weiter gezeigt, daß man der organischen Lösung zusätzlich noch eine feste organische Carbonsäure zugeben kann.

Die unter Verwendung dieser Stabilisatoren hergestellten Keton-Papiere sind weiß. Mit ihnen können somit schon Spuren von Keton-Körpern nachgewiesen werden. So liegt die Empfindlichkeitsgrenze für Acetessigsäure bei 3-4 mg%, so daß physiologische Menge an Acetessigsäure gerade nicht mehr registriert werden. Die Stabilität der Papiere ist sehr gut, Bei einer Lagerung während 48 Stunden bei Raumtemperatur und 60 % relativer Luftfeuchtigkeit verlieren die Papiere ihre weiße Farbe nicht. Dagegen sind Papiere, die gemäß DE-PS 21 48 125 erhalten werden, unter den gleichen Bedingungen schon nach 60 Minuten stark verfärbt. Die stabilisierten Papiere können auch ohne besondere Vorsichtsmaßnahmen konfektioniert werden. Dabei sind sie elastisch und brechen nicht.

Die folgenden Beispiele sollen die vorstehend beschriebene Erfindung erläutern.

### Beispiel 1

Imprägnierlösung 1 :
80 g Glycin
17 g Natriumhydroxid
100 ml Wasser
pH-Wert auf 10,0 korrigieren.

Imprägnierlösung 2 :
1 g Natriumnitroprussid
1,6 g 18-crown-6
40 ml Methanol
20 ml Dimethylformamid.

Papier Schleicher und Schüll 2316 wird zunächst mit der Imprägnierlösung 1 behandelt und anschließend bei 80 °C so lange getrocknet, bis das Papier eine Restfeuchte unter 3 % hat. Anschließend erfolgt die zweite Imprägnierung. Das erhaltene Papier ist farblos und weist die früher beschriebenen positiven Eigenschaften auf.

### Beispiel 2

Imprägnierlösung 1 : entsprechend Beispiel 1

Imprägnierlösung 2 :
0,5 g Natriumnitroprussid
0,3 g Dibenzo-18-crown-6
40 ml Methanol
20 ml Propanol.

Verarbeitung analog Beispiel 1. Auch diese Papiere sind besonders lagerstabil.

### Beispiel 3

Das Beispiel 2 wird wiederholt. Dem organischen Lösemittel werden zusätzlich 3 g Citronensäure zugegeben. Das Ergebnis ist wieder ein stabiles Papier.

### Beispiel 4

Imprägnierlösung 1 :
80 g Glycin
17 g Natriumhydroxid
2,5 g Kryptofix(R) 222 (Merck 10647)
100 ml Wasser
pH-Wert auf 10,0 korrigieren.

Imprägnierlösung 2 :
0,5 g Natriumnitroprussid
1,0 g Citronensäure
40 ml Methanol
20 ml propanol

Auch dieses Papier weist die geschilderten Vorteile auf.

**Patentansprüche** (für die Vertragsstaaten : BE, CH, DE, FR, GB, IT, LI, LU, NL, SE)

1. Ein diagnostisches Mittel zum Nachweis von Ketonkörpern in Flüssigkeiten bestehend aus einer Trägermatrix, einem Puffer, einer Aminosäure, Natriumnitroprussid, gegebenenfalls einer organischen Carbonsäure, und einem Stabilisator, dadurch gekennzeichnet, daß der Stabilisator eine Verbindung der Formel I

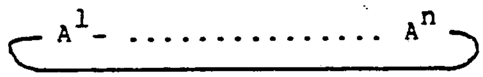

(I)

in welcher n eine Zahl von 2 bis 10 ist, und worin ein A-Glied

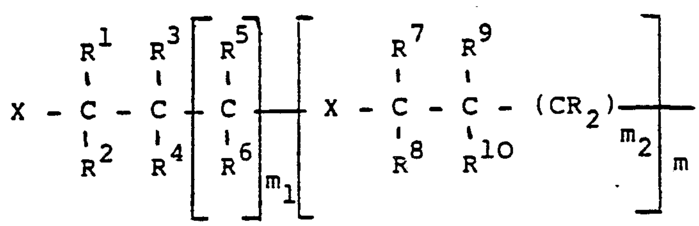

bedeutet, worin

X ein Sauerstoffatom, ein Schwefelatom oder NH,

R bis $R^{10}$ je ein Wasserstoffatom,

$m_1$ und $m_2$ eine Zahl von 0 bis 4 und

m die Zahl 0 oder 1, wobei jedoch mindestens vier X-Reste in der Verbindung vorhanden sein müssen, ist und außerdem

$R^3$, $R^4$, $R^9$ und $R^{10}$ $C_{1-3}$-Alkyl

$R^1$ und $R^3$ oder $R^7$ und $R^9$ zusammen $CH_2CH_2CH_2CH_2$ oder CH=CH—CH=CH und $—R^2$ und $—R^4$ oder

$—R^8$ und $—R^{10}$ zusammen eine Bindung und $R^5$, $R^6$, $R^7$ und $R^8$ zusammen CH—CH, wobei $m_1 = 1$ ist, (sowie $R^1$ und $R^2$ sowie $R_2$ mit den entsprechenden R-Resten der nicht gezeichneten Nachbarglieder A, wobei $m_2 = 1$ ist, zusammen CH—CH) sein können

oder eine Verbindung der Formel II ist

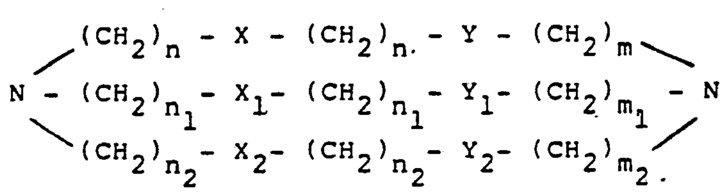

(II)

in welcher

n, $n_1$ und $n_2$ die Zahlen 2 oder 3,

m, $m_1$ und $m_2$ eine Zahl von 0 bis 3,

X, $X_1$, $X_2$, Y, $Y_1$ und $Y_2$ ein Sauerstoff- oder Schwefelatom oder

—Y—, $—Y_1—$ und $—Y_2—$ eine Bindung, in welchem Fall m, $m_1$ beziehungsweise $m_2 = 0$ ist,

bedeutet.

2. Ein Mittel nach Anspruch 1, dadurch gekennzeichnet, daß der Stabilisator eine Verbindung der Formel I ist, in welcher X ein Sauerstoffatom oder NH, $m_1$ und $m_2$ die Zahl 0 oder 1, $R^5$ und $R^6$, falls $m_1$ gleich 1 ist, je ein Wasserstoffatom bedeuten und die übrigen Symbole die oben angegebene Bedeutung haben.

3. Ein Mittel nach Anspruch 1, dadurch gekennzeichnet, daß der Stabilisator eine Verbindung der Formel I und den Bedeutungen der Symbole

n : die Ziffer 2 oder 3

X : ein Sauerstoffatom

$m_1$, $m_2$ : Null

$R^1$-$R^4$ : ein Wasserstoffatom

$R^7$-$R^{10}$ : ein Wasserstoffatom

$R^1$ und $R^3$ : zusammen CH=CH—CH=CH oder $CH_2—CH_2—CH_2—CH_2$

$R^2$ und $R^4$ : zusammen ein Bindung.

m : 0 oder 1

ist.

4. Ein Mittel nach Anspruch 1, dadurch gekennzeichnet, daß der Stabilisator 12-crown-4, 15-crown-5, 18-crown-6, Dibenzo-18-crown-6, Dicyclohexano-18-crown-6, 1,4,7,10,13,16-Hexaaza-cyclooctadecan-trisulfat, 4,7,13,16,21,24-Hexaoxa-1,10-diazabicyclo-(8,8,8)-hexacosan, 4,7,13,16,21-Pentaoxa-1,10-diazabicyclo-(8,8,5)-tricosan oder 4,7,13,18-Tetraoxa-1,10-diazabicyclo-(8,5,5)-eicosan ist.

5. Mittel nach Anspruch 1, dadurch gekennzeichnet, daß der Stabilisator die Kronenverbindung Dibenzo-18-crown-6 oder 18-crown-6 ist.

6. Verfahren zur Herstellung eines diagnostischen Mittels zum Nachweis von Ketonkörpern in Flüssigkeiten, dadurch gekennzeichnet, daß auf einer Trägermatrix ein alkalischer Puffer, eine Aminosäure, Natriumnitroprussid, gegebenenfalls eine organische Carbonsäure, und eine Verbindung der Formel I oder II aus Anspruch 1 aufgrebracht wird.

7. Verwendung einer Verbindung der Formel I oder II aus Anspruch 1 in einem diagnostischen Mittel zum Nachweis von Ketonkörpern in Flüssigkeiten.

**Patentanspruch** (für den Vertragsstaat AT)

Ein Verfahren zum Nachweis von Ketonkörpern in Flüssigkeiten, dadurch gekennzeichnet, daß ein diagnostisches Mittel bestehend aus einer Trägermatrix, einem Puffer, einer Aminosäure, Natriumnitroprussid, gegebenenfalls einer organischen Carbonsäure, und einer Verbindung der Formel I

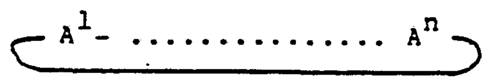

(I)

in welcher n eine Zahl von 2 bis 10 ist, und worin ein A-Glied

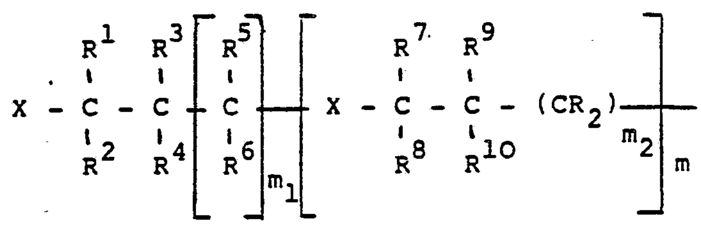

bedeutet, worin

X ein Sauerstoffatom, ein Schwefelatom oder NH,
R bis $R^{10}$ je ein Wasserstoffatom,
$m_1$ und $m_2$ eine Zahl von 0 bis 4 und
m die Zahl 0 oder 1, wobei jedoch mindestens vier X-Reste in der Verbindung vorhanden sein müssen, ist und außerdem
$R^3$, $R^4$, $R^9$ und $R^{10}$ $C_{1-3}$-Alkyl
$R^1$ und $R^3$ oder $R^7$ und $R^9$ zusammen $CH_2CH_2CH_2CH_2$ oder CH=CH—CH=CH und —$R^2$ und —$R^4$ oder
—$R^8$ und —$R^{10}$ zusammen eine Bindung und $R^5$, $R^6$, $R^7$ und $R^8$ zusammen CH—CH,
wobei $m_1$ = 1 ist, (sowie $R^1$ und $R^2$ sowie $R_2$ mit den entsprechenden R-Resten der nicht gezeichneten Nachbarglieder A, wobei $m_2$ = 1 ist, zusammen CH—CH) sein können
oder eine Verbindung der Formel II ist

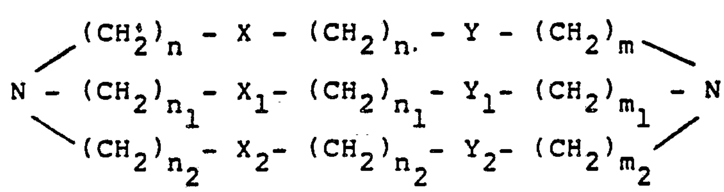

(II)

in welcher
n, $n_1$ und $n_2$ die Zahlen 2 oder 3,
m, $m_1$ und $m_2$ eine Zahl von 0 bis 3,
X, $X_1$, $X_2$, Y, $Y_1$ und $Y_2$ ein Sauerstoff- oder Schwefelatom oder
—Y—, —$Y_1$— und —$Y_2$— eine Bindung, in welchem Fall m, $m_1$ beziehungsweise $m_2$ = 0 ist,
bedeutet, als Stabilisator mit der zu prüfenden Flüssigkeit in Kontakt gebracht wird.

**Claims** (for the Contracting States : BE, CH, DE, FR, GB, IT, LI, LU, NL, SE)

1. A diagnostic agent useful in the detection of ketone bodies in fluids, which consists of a carrier matrix, that has been impregnated with a buffer, an amino acid and sodium nitroprusside in an organic solvent, said diagnostic agent being characterized by a content of a compound of formula I

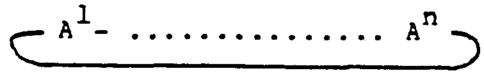 (I)

wherein

n is an integer of from 2 to 10 and an A member represents the following structure,

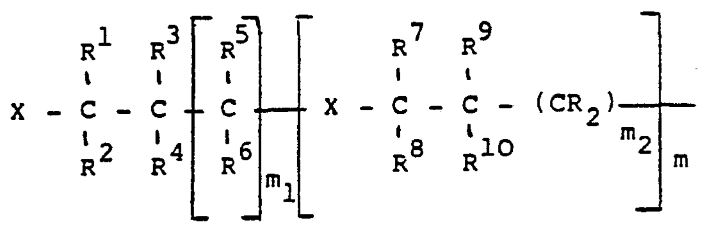

wherein

X is oxygen, sulfur or NH,

R to $R^{10}$ each are hydrogen,

$m_1$ and $m_2$ each represent an integer of from 0 to 4 and

m is zero or 1, with the proviso that at least four X radicals have to to present in the compound, and moreover

$R^3$, $R^4$, $R^9$ and $R^{10}$ each may be $C_{1-3}$ alkyl,

$R^1$ and $R^3$ or $R^7$ and $R^9$ may form together a $CH_2CH_2CH_2CH_2$ or a CH=CH—CH=CH group and —$R^2$ and —$R^4$ or —$R^8$ and —$R^{10}$ may form together a bond and

$R^5$, $R^6$, $R^7$ and $R^8$ may form together a CH—CH group, in which case $m_1 = 1$ (and $R^1$ and $R^2$ as well as $R_2$ and the corresponding R radicals of the adjacent A members that are not shown in formula I, may form together a CH—CH group, in which case $m_2 = 1$),

or a compound of the formula II

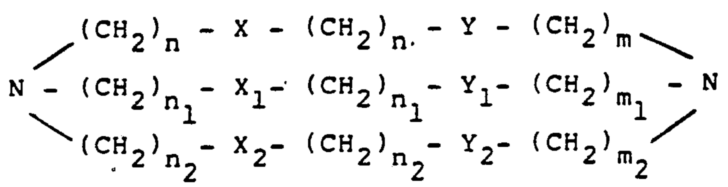 (II)

wherein

n, $n_1$ and $n_2$ each are 2 or 3,

m, $m_1$ and $m_2$ are an integer of from 0 to 3,

X, $X_1$, $X_2$, Y, $Y_1$ and $Y_2$ each are oxygen or sulfur or —Y—, —$Y_1$— and —$Y_2$— each represent a bond, in which case m, $m_1$, and $m_2$, respectively, are 0.

2. An agent as claimed in claim 1, wherein the stabilizer is a compound of formula I wherein X is oxygen or NH, $m_1$ and $m_2$ each are zero or 1, $R^5$ and $R^5$ each are hydrogen, if $m_1$ is 1 and the other symbols have the above-mentioned meanings.

3. An agent as claimed in claim 1, wherein the stabilizer is a compound of formula I, the symbols whereof having the following meanings :

n is an integer of from 2 to 3,

X is oxygen,

$m_1$, $m_2$ is zero,

$R^1$-$R^4$ each are hydrogen,

$R^7$-$R^{10}$ each are hydrogen,

$R^1$ and $R^3$ represent together a CH=CH—CH=CH or $CH_2$—$CH_2$—$CH_2$—$CH_2$ group,

$R^2$ and $R^4$ represent together a bond and

m is zero or 1.

4. An agent as claimed in claim 1, wherein the stabilizer is 12-crown-4, 15-crown-5, 18-crown-6, dibenzo-18-crown-6, dicyclohexano-18-crown-6, 1,4,7,10,13,16-hexa-aza-cyclooctadecane trisulfate or

4,7,13,16,21,24-hexa-oxa-1,10-diazabicyclo-(8,8,8)-hexaosane, 4,7,13,16,21-pentaoxa-1,10-diazabicyclo-(8,8,5)-tricosane or 4,7,13,18-tetraoxa-1,10-diazabicyclo-(8,5,5)-eicosane.

5. An agent as claimed in claim 1, wherein the stabilizer is dibenzo-18-crown-6 or 18-crown-6.

6. A process for the preparation of a diagnostic agent useful in the detection of ketone bodies in fluids, which comprises applying to a carrier matrix an alkaline buffer, an amino acid, sodium nitroprusside, optionally an organic carboxylic acid, and a compound of formula I or II of claim 1.

7. Method of using a compound of formula I or II of claim 1 in a diagnostic agent useful in the detection of ketone bodies in fluids.

**Claim** (for the Contracting State AT)

A process for detecting ketone bodies in fluids, which comprises contacting the fluid to be tested with a diagnostic agent comprising a carrier matrix, a buffer, an amino acid, sodium nitroprusside, optionally an organic carboxylic acid, and a compound of formula I

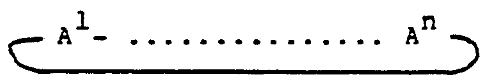

(I)

wherein

n is an integer of from 2 to 10 and an A member represents the following structure,

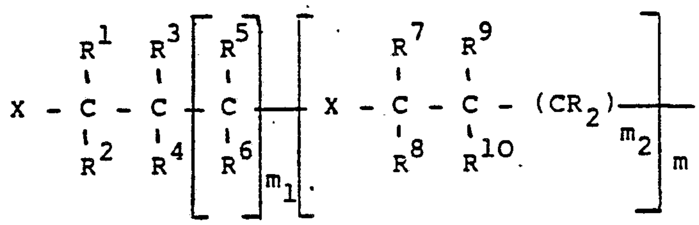

wherein

X is oxygen, sulfur or NH,

R to $R^{10}$ each are hydrogen,

$m_1$ and $m_2$ each represent an integer of from 0 to 4 and

m is zero or 1, with the proviso that at least four X radicals have to to present in the compound, and moreover

$R^3$, $R^4$, $R^9$ and $R^{10}$ each may be $C_{1-3}$ alkyl,

$R^1$ and $R^3$ or $R^7$ and $R^9$ may form together a $CH_2CH_2CH_2CH_2$ or a CH=CH—CH=CH group and —$R^2$ and —$R^4$ or —$R^8$ and —$R^{10}$ may form together a bond and

$R^5$, $R^6$, $R^7$ and $R^8$ may form together a CH—CH group, in which case $m_1 = 1$ (and $R^1$ and $R^2$ as well as $R_2$ and the corresponding R radicals of the adjacent A members that are not shown in formula I, may form together a CH—CH group, in which case $m_2 = 1$),

or a compound of the formula II

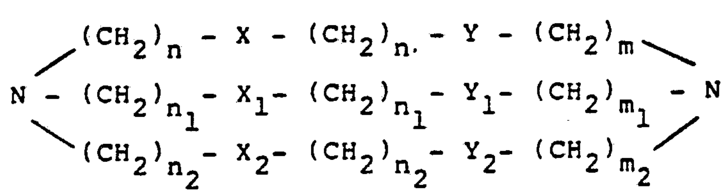

(II)

wherein

n, $n_1$ and $n_2$ each are 2 or 3,

m, $m_1$ and $m_2$ are an integer of from 0 to 3,

X, $X_1$, $X_2$, Y, $Y_1$ and $Y_2$ each are oxygen or sulfur or —Y—, —$Y_1$— and —$Y_2$— each represent a bond, in which case m, $m_1$ and $m_2$, respectively, are 0 as a stabilizer.

**Revendications** (pour les Etats contractants : BE, CH, DE, FR, GB, IT, LI, LU, NL, SE).

1. Moyen de diagnostic pour la détection des corps cétoniques dans les liquides constitué d'une matrice support, d'un tampon, d'un aminoacide, de nitroprussiate de sodium, éventuellement d'un acide

carboxylique organique et d'un stabilisant, caractérisé en ce que le stabilisant est un composé de formule I

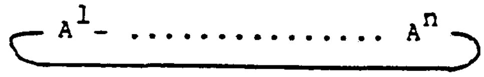 (I)

dans laquelle n est un nombre de 2 à 10 et un motif A représente

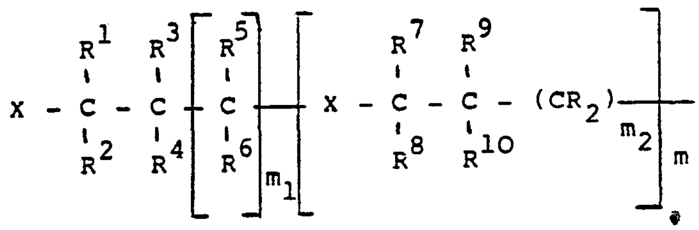

où

X est un atome d'oxygène, un atome de soufre ou NH,

R à $R^{10}$ sont chacun un atome d'hydrogène,

$m_1$ et $m_2$ sont des nombres de 0 à 4 et

m est égal à 0 ou 1, quatre radicaux X au moins devant être présents dans le composé, et en outre $R^3$, $R^4$, $R^9$ et $R^{10}$ peuvent être des groupes alkyle en $C_{1-3}$

$R^1$ et $R^3$ ou $R^7$ et $R^9$ peuvent former ensemble $CH_2CH_2CH_2CH_2$ ou CH=CH—CH=CH et —$R^2$ et —$R^4$ ou —$R^8$ et —$R^{10}$ peuvent former une liaison entre eux

et $R^5$, $R^6$, $R^7$ et $R^8$ peuvent former ensemble CH—CH,

$m_1$ étant égal à 1 (et $R^1$ et $R^2$ ainsi que $R_2$ avec les radicaux R correspondants des motifs voisins A non représentés formant ensemble CH—CH, $m_2$ étant égal à 1) ou un composé de formule II :

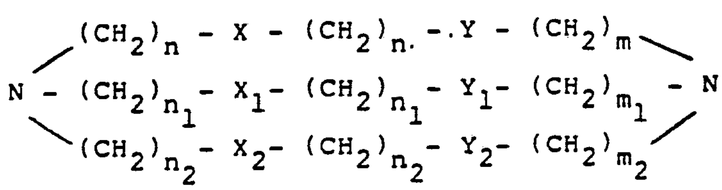 (II)

dans laquelle

n, $n_1$ et $n_2$ sont égaux à 2 ou 3,

m, $m_1$ et $m_2$ sont des nombres de 0 à 3,

X, $X_1$, $X_2$, Y, $Y_1$ et $Y_2$ sont un atome d'oxygène ou de soufre ou

—Y—, —$Y_1$— et —$Y_2$— représentent une liaison, auquel cas m, $m_1$ et respectivement $m_2 = 0$.

2. Moyen selon la revendication 1, caractérisé en ce que le stabilisant est un composé de formule I dans laquelle X est un atome d'oxygène ou NH, $m_1$ et $m_2$ sont égaux à 0 ou 1, $R^5$ et $R^6$, au cas où $m_1$ est égal à 1, représentent chacun un atome d'hydrogène, et les autres symboles ont la signification indiquée ci-dessus.

3. Moyen selon la revendication 1, caractérisé en ce que le stabilisant est un composé de formule I, les symboles ayant les significations suivantes

n : 2 ou 3

X : un atome d'oxygène

$m_1$, $m_2$ : zéro

$R^1$ à $R^4$ : un atome d'hydrogène

$R^7$ à $R^{10}$ : un atome d'hydrogène

$R^1$ et $R^3$ : ensemble CH=CH—CH=CH ou $CH_2$—$CH_2$—$CH_2$—$CH_2$

$R^2$ et $R^4$ : ensemble une liaison

m : 0 ou 1.

4. Moyen selon la revendication 1, caractérisé en ce que le stabilisant est le 12-crown-4, le 15-crown-5, le 18-crown-6, le dibenzo-18-crown-6, le dicyclohexano-18-crown-6, le 1,4,7,10,13,16-hexa-aza-cyclooctadécanetrisulfate, le 4,7,13,16,21,24-hexa-oxa-1,10-diazabicyclo-(8,8,8)-hexacosane, le 4,7,13,16,21-pentaoxa-1,10-diazabicyclo-(8,8,5)-tricosane ou le 4,7,13,18-tétraoxa-1,10-diazabicyclo-(8,5,5)-eicosane.

5. Moyen selon la revendication 1, caractérisé en ce que le stabilisant est le composé à structure en couronne dibenzo-18-crown-6 ou 18-crown-6.

6. Procédé de préparation d'un moyen de diagnostic pour la détection des corps cétoniques dans les liquides, caractérisé en ce que l'on applique sur une matrice support un tampon alcalin, un aminoacide, du nitroprussiate de sodium, éventuellement un acide carboxylique organique et un composé de formule I ou II définis à la revendication 1.

7. Utilisation d'un composé de formule I ou II défini à la revendication 1 dans un moyen de diagnostic pour la détection des corps cétoniques dans les liquides.

**Revendication** (pour l'Etat contractant AT)

Procédé pour la détection de corps cétoniques dans les liquides, caractérisé en ce que l'on met en contact avec le liquide à analyser un moyen de diagnostic consistant en une matrice support, un tampon, un aminoacide, du nitroprussiate de sodium, éventuellement un acide carboxylique organique et, comme stabilisant, un composé de formule I

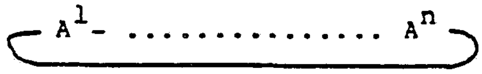 (I)

dans laquelle n est un nombre de 2 à 10 et le motif A représente

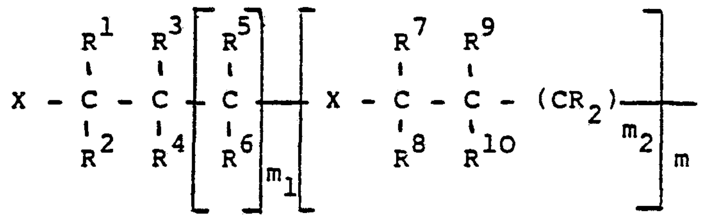

où

X est un atome d'oxygène, un atome de soufre ou NH,
R à $R^{10}$ sont chacun un atome d'hydrogène,
$m_1$ et $m_2$ sont des nombres de 0 à 4 et
m est égal à 0 ou 1, quatre radicaux X au moins devant être présents dans le composé, et en outre $R^3$, $R^4$, $R^9$ et $R^{10}$ peuvent être des groupes alkyle en $C_{1-3}$
$R^1$ et $R^3$ ou $R^7$ et $R^9$ peuvent former ensemble $CH_2CH_2CH_2CH_2$ ou CH=CH—CH=CH et $—R^2$ et $—R^4$ ou $—R^8$ et $—R^{10}$ peuvent former une liaison entre eux
et $R^5$, $R^6$, $R^7$ et $R^8$ peuvent former ensemble CH—CH,
$m_1$ étant égal à 1 (et $R^1$ et $R^2$ ainsi que $R_2$ avec les radicaux R correspondants des motifs voisins A non représentés formant ensemble CH—CH, $m_2$ étant égal à 1)
ou un composé de formule II :

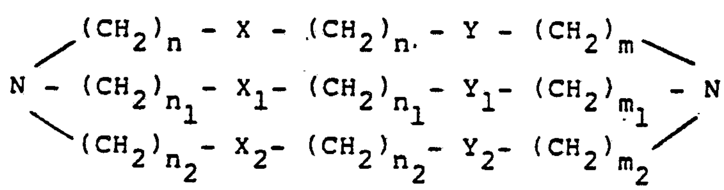 (II)

dans laquelle
$n$, $n_1$ et $n_2$ sont égaux à 2 ou 3,
$m$, $m_1$ et $m_2$ sont des nombres de 0 à 3,
X, $X_1$, $X_2$, Y, $Y_1$ et $Y_2$ sont un atome d'oxygène ou de soufre ou
—Y—, $—Y_1—$ et $—Y_2—$ représentent une liaison, auquel cas m, $m_1$ et respectivement $m_2 = 0$.